Europäisches Patentamt

European Patent Office

Office européen des brevets

(11) Publication number: **0 139 881**
**B1**

## (12) EUROPEAN PATENT SPECIFICATION

(45) Date of publication of patent specification: **10.05.89**

(21) Application number: **84108609.3**

(22) Date of filing: **20.07.84**

(51) Int. Cl.⁴: **A 61 K 9/10,** A 61 K 9/52,
A 61 K 31/74 // A61K31/485,
A61K31/135

(54) Liquid prolonged release pharmaceutical formulations containing ionic constituents.

(30) Priority: **16.09.83 US 532864**

(43) Date of publication of application:
**08.05.85 Bulletin 85/19**

(45) Publication of the grant of the patent:
**10.05.89 Bulletin 89/19**

(84) Designated Contracting States:
**AT BE CH DE FR GB IT LI LU NL SE**

(56) References cited:
**AT-B- 279 802
DE-B-2 246 037
US-A-2 990 332
US-A-3 138 525
US-A-4 221 778**

(73) Proprietor: **PENNWALT CORPORATION
Pennwalt Building Three Parkway
Philadelphia Pennsylvania 19102 (US)**

(72) Inventor: **Sheumaker, Jerry L.
44 Delemere Blvd.
Fairport New York 14450 (US)**

(74) Representative: **Kraus, Walter, Dr. et al
Patentanwälte Kraus, Weisert & Partner
Thomas-Wimmer-Ring 15
D-8000 München 22 (DE)**

## EP 0 139 881 B1

**Description**

The present invention relates to a liquid form of prolonged continuous release pharmaceutical formulations containing ionic components. More particularly, the invention relates to such formulations wherein the prolonged release of the active drug is accomplished by providing a semi-permeable coating around discrete, minute, ion exchange resin particles with which the drug component has been reacted to from an insoluble drug-resin complex. The semi-permeable coating creates a diffusion barrier, the thickness of which can be adjusted to provide the desired level of retardation of drug availability in the gastrointestinal tract over a period of time. The preparation of typical coated, insoluble drug-polymer complexes is disclosed in detail in US—A—4,221,778.

Drugs utilizing the delivery system described in US—A—4,221,778 may be administered in either capsule or liquid suspension form. The feasibility of liquid dosage is a particularly important advantage of the invention described in US—A—4,221,778 because most commercial sustained release systems require solid coatings whose rate of solution controls drug availability, thereby precluding administration in liquid form. Nevertheless, in attempts to provide formulations using the coated, insoluble drug-resin complexes described in US—A—4,221,778 it was observed that the presence of ionic substances in the formulation at times disturbed the expected dissolution profile for the coated drug-resin complex. The problem was observed to be paricularly accute when attempts were made to provide formulations of combination type drugs where each drug was ionic and of the same ionic charge and where at least one drug was present in the coated, drug-resin complex form while at least one other (usually present in very minor amounts) was present in its ionic form. The present invention addresses this problem by providing that substantially all ionic components in a formulation of the sustained release drug type described in US—A—4,221,778 which component bears the same ionic charge as the drug on the coated, drug-resin complex be present as a resin complex.

In addition to US—A—4,221,778 mentioned above, various coated resins and drug-resin complexes have been reported (e.g., in US—A—3,138,525; 3,499,960 and 3,594,470; BE—A—729,827; DE—A—2,246,037; and Brodkins et al, Journal of Pharmaceutical Science, Vol. 60, pages 1523—1527, 1971), but none suggest a solution to the problem disclosed herein.

The present invention provides a pharmaceutical composition containing a drug which can be ionic, selected from pseudoephredine, codeine, hydrocodone, phenylpropanolamine, dextramorphan and ephedrine, when the drug is present as a coated drug-resin complex suspended in a liquid carrier, which is characterized in that the carrier also contains chlorpheniramine as a second ionic component bearing the same charge as the coated complexed drug, the second ionic component being present as an uncoated resin complex. While applicant does not wish to be bound by any theory of operation, it is postulated that when the second ionic component of like charge to the coated drug is present in a formulation, the unbound ion penetrates the diffusion barrier and, to some degree, exchanges upon the resin particle with the drug previously bound thereby causing an increase in the amount of unbound drug in the formulation as well as producing a change in the dissolution profile (i.e. the amount of drug in solution versus time) of the previously bound drug; solution of the problem is accomplished by binding the second ionic component to its own resin.

Pursuant to US—A—4,221,778 it has been found that a selective, prolonged continuous release of pharmacologically active drugs, under conditions such as those encountered in the gastrointestinal tract, can be achieved by the application of a diffusion barrier coating to an ion exchange drug-resin complex particle which has been treated with a solvating agent; the present invention provides an improvement whereby prolonged release formulations from coated drugs of the type as described in US—A—4,221,778 can be prepared under circumstances wherein a component of the formulation contains a second ionic substance (e.g. a combination drug, a dye or a dispersing agent) bearing the same ionic charge as the drug on the drug-resin complex by employing the second ionic substance in the ion form of an exchange resin complex. The manufacture of a formulation of any drug for liquid dosage usage requires that the final formulation have the drug dissolved or suspended in a liquid that is pleasing to the eye and taste, possess extended shelf-life stability, and exhibit no change in active drug dosage level over a period of time. Thus, to prepare a liquid formulation of any type drug, including one of the type as described in US—A—4,221,778 it is necessary to employ extenders such as water or syrup, and to add flavors, sweeteners, thickening agents or dyes. Since the presence of these non-biologically active components in contact with the semi-permeable membranes taught by US—A—4,221,778 will often have some effect on the ability of the active drug to escape in the digestive system from the ion complex upon which it is held it is essential that the dissolution profile of the particular drug system (i.e. the amount of the drug released over stated periods of time) be measured in the formulation in which one intends to administer a particular dosage. From time to time in the transition of the products as described in US—A—4,221,778 from the laboratory to commercail embodiments, erratic results have been obtained in the dissolution profile of the formulation versus the dissolution profile of the same drug in water. It was determined that one source of such erratic results was the presence of ionic substances bearing the same ionic charge as the sustained release drug present in the formulation as a coated drug-resin complex; the presence of ionic substances of opposite charge appear to have no effect on the expected dissolution rate. Pursuant to the present invention it was found that if the second ionic substance of the same charge is bound upon a resin complex

2

of its own, the erratic results are largely eliminated. Resins suitable for binding the second ionic component may be any of those previously disclosed in US—A—4,221,778.

Obviously, the second ionic material need not be coated with the water-permeable diffusion barrier coating taught in US—A—4221778.

The following examples are cited to illustrate the invention.

### Example I

A Raghunathan type prolonged release coated, drug resin complex was prepared from pseudoephedrine generally following Example 20 of US—A—4,221,778. A formulation was prepared from 49.7 g. of the coated, drug resin complex using approximately 600 ml. of xanthan gum, pregelatinized starch and water as the suspending agent. Also included in the composition was an aqueous solution of a dye, 400 g. of granulated sugar and corn syrup, a wetting agent (propylene glycol), and, in very small quantities, a preservative and flavorings. A sample of this formulation was withdrawn and tested in various intervals to determine the availability of the pseudoephedrine under conditions which would be encountered in the digestive system. The resutls obtained appear as Column A in Table 1.

In an attempt to prepare a combination drug of pseudoephedrine and chlorpheniramine, 1.2 g. of chlorpheniramine maleate was added to a formulation substantially identical to the above (except for dyes and flavorings). A sample of the combination drug formulation was analyzed to provide the dissolution profile of pseudoephedrine which is reported in Table I, Column B. A comparison of the results shown in Columns A and B discloses a substantial decrease in the availability of pseodoephedrine during the first hour and a half after dosage and a substantial increase in availability thereafter as compared with the results obtained with pseudoephedrine alone.

In a third embodiment the same amount of chlorpheniramine maleate as used above was added in the form of a resin complex, using the same resin (Amberlite XE—69)® employed to complex the pseudoephedrine. A small sample examined for the dissolution profile of pseudoephedrine produced the result shown in Column C of Table I. It will be obvious from the table that when the second ionic component is added in the form of the resin complex there is substantially no disturbing effect upon the dissolution profile of the ephedrine.

### TABLE I

| Interval (Hrs.) | Pseudoephedrine Availability (%) | | |
|---|---|---|---|
| | A | B | C |
| $\frac{1}{2}$ | 49.9 | 23.1 | 51.6 |
| 1 | 53.4 | 28.7 | 53.5 |
| 3 | 66.5 | 78.8 | 64.9 |
| 6 | 73.9 | 87.8 | 72.3 |

### Example II

Not only is the dissolution profile of the coated drug resinate changed when a second drug is present in ionic from in the same formulation versus the same combination when the second drug is in the form of a drug resinate, but the dissolution rate of the second drug also varies under such circumstances. Thus from the dissolution results of Table II, wherein codeine (as a coated drug resinate) in substantially the same formulation suspension as taught in Example I is present with chlorpheniramine, (in ionic form in Run E but present in drug resinate from in Run D) it can be seen that the presence of the chlorpheniramine (second component) in its ionic from retards the availability of each of the drugs over the period of observation.

### TABLE II

| Interval (Hrs.) | Codeine | | Chlorpheniramine | |
|---|---|---|---|---|
| | $D^{XX}$ | $E^*$ | $D^{XX}$ | $E^*$ |
| 0.5 | 31.2 | 24.0 | 48.7 | 34.9 |
| 1.0 | 35.4 | 29.5 | 57.4 | N.M. |
| 3.0 | 44.0 | 40.2 | 69.7 | 58.9 |
| 6.0 | 49.9 | 48.1 | 77.0 | N.M. |

N.M. indicates value not measured.
* Chlorpheniramine present in ionic form.
$^{XX}$ Chlorpheniramine present in drug-resinate form.

In the embodiment of this example the coated codeine-resinate (prepared in the manner taught for pseudoephedrine in Example I) was used in an amount of 17.7 gm.; the chlorpheniramine was present in an amount of 0.80 gm.. In the resinated form the chlorpheniramine was bound to Amberlite XE—69® resin.

Another typical combination to which the present invention is applicable is hydrocodone and chlorpheniramine where the hydrocodone is present in the coated, hydrocodone-resinate form.

While the present invention is of particular importance in the formulation of ionic type combination drugs, it will be obvious that it can be employed to control the erratic dissolution characteristics of single ionic drugs from formulations containing any extraneous ionic material. For instance, the erratic dissolution problem can be caused by the presence of a dye of appropriate ionic charge; pursuant to the present invention the dye would be complexed with a resin prior to addition to the formulation. The types of medications that could be useful in combined form in the present invention are many, thus combinations of any of the following are possible: analgesics, decongestants, anorexics, antiarthritics, antiasthmas, antibiotics, antidepressants, antihypertensives, antiinflammatories, antipsychotics, antispasmotics, anticholinergics, and muscle relaxants.

### Claims for the Contracting States: BE CH DE FR GB IT LI LU NL SE

1. A pharmaceutical composition containing a drug which can be ionic selected from pseudoephedrine, codeine, hydrocodone, phenylpropanolamine, dextramorphan and ephedrine, wherein the drug is present as a coated drug-resin complex suspended in a liquid carrier, characterized in that the carrier also contains chlorpheniramine as a second ionic component bearing the same charge as the coated complexed drug, the second ionic component being present as an uncoated resin complex.

2. The pharmaceutical composition of Claim 1 wherein the drug which can be ionic is pseudoephedrine.

3. The pharmaceutical composition of Claim 1 wherein the drug which can be ionic is codeine.

4. The pharmaceutical composition of Claim 1 wherein the drug which can be ionic is hydrocodone.

### Claims for the Contracting State: AT

1. A process for preparing a sustained release pharmaceutical composition containing a drug which can be ionic selected from pseudoephedrine, codeine, hydrocodone, phenylpropanolamine, dextramorphan and ephedrine, wherein the drug is present as a coated drug-resin complex suspended in a liquid carrier, comprising the steps of (a) forming a liquid suspension of said coated drug-resin complex in a first vessel, (b) forming a liquid suspension of an uncoated chlorpheniramine-resin complex in a second vessel, and thereafter (c) combining suspensions (a) and (b).

2. A process for preparing a sustained release pharmaceutical composition containing a drug which can be ionic selected from pseudoephedrine, codeine, hydrocodone, phenylpropanolamine, dextramorphan and ephedrine, wherein the drug is present as a coated drug-resin complex suspended in a liquid carrier, which comprises suspending both the coated drug-resin complex and the uncoated chlorpheniramine-resin complex in a liquid.

3. The process of Claim 1 or 2 wherein the drug which can be ionic is pseudoephedrine.

4. The process of Claim 1 or 2 wherein the drug which can be ionic is codeine.

5. The process of Claim 1 or 2 wherein the drug which can be ionic is hydrocodone.

### Patentansprüche für die Vertragsstaaten: BE CH DE FR GB IT LI LU NL SE

1. Pharmazeutisches Präparat, enthaltend einen Wirkstoff, der ionogen sein kann und aus Pseudoephedrin, Codein, Hydrocodon, Phenylpropanolamin, Dextramorphan und Ephedrin ausgewählt ist, wobei der Wirstoff als beschichteter Wirkstoff-Harz-Komplex, suspendiert in einem flüssigen Träger, vorliegt, dadurch gekennzeichnet, daß der Träger auch Chlorpheniramin als zweite ionogene Komponente, die die gleiche Ladung wie der beschichtete, in Komplexform überführte Wirkstoff trägt, enthält, wobei die zweite ionogene Komponente als unbeschichteter Harzkomplex vorhanden ist.

2. Pharmazeutisches Präparat nach Anspruch 1, dadurch gekennzeichnet, daß der Wirkstoff, der ionogen sein kann, Pseudoephedrin ist.

3. Pharmazeutisches Präparat nach Anspruch 1, dadurch gekennzeichnet, daß der Wirkstoff, der ionogen sein kann, Codein ist.

4. Pharmazeutisches Präparat nach Anspruch 1, dadurch gekennzeichnet, daß der Wirkstoff, der ionogen sein kann, Hydrocodon ist.

### Patentansprüche für den Vertragsstaat: AT

1. Verfahren zur Herstellung eines pharmazeutischen Retardpräparats, enthaltend einen Wirkstoff, der ionogen sein kann und aus Pseudoephedrin, Codein, Hydrocodon, Phenylpropanolamin, Dextramorphan und Ephedrin ausgewählt ist, wobei der Wirkstoff als beschichteter Wirkstoff-Harz-Komplex, suspendiert in einem flüssigen Träger, vorliegt, gekennzeichnet durch die Stufen (a) Bilden einer flüssigen Suspension des genannten beschichteten Wirkstoff-Harz-Komplexes in einem ersten Gefäß, (b) Bilden einer flüssigen Suspension eines unbeschichteten Chlorpheniramin-Harz-Komplexes in einem zweiten Gefäß und danach (c) Kombinieren der Suspensionen (a) und (b).

2. Verfahren zur Herstellung eines pharmazeutischen Retardpräparats, enthaltend einen Wirkstoff, der ionogen sein kann und aus Pseudoephedrin, Codein, Hydrocodon, Phenylpropanolamin, Dextramorphan und Ephedrin ausgewählt ist, wobei der Wirkstoff als beschichteter Wirkstoff-Harz-Komplex, suspendiert in einem flüssigen Träger, vorliegt, dadurch gekennzeichnet, daß man sowohl den beschichteten Wirkstoff-Harz-Komplex als auch den unbeschichteten Chlorpheniramin-Harz-Komplex in einer Flüssigkeit suspendiert.

3. Verfahren nach Anspruch 1 oder 2, dadurch gekennzeichnet, daß der Wirkstoff, der ionogen sein kann, Pseuodoephedrin ist.

4. Verfahren nach Anspruch 1 oder 2, dadurch gekennzeichnet, daß der Wirkstoff, der ionogen sein kann, Codein ist.

5. Verfahren nach Anspruch 1 oder 2, dadurch gekennzeichnet, daß der Wirkstoff, der ionogen sein kann, Hydrocodon ist.

**Revendications pour les Etats contractants: BE CH DE FR GB IT LI LU NL SE**

1. Composition pharmaceutique contenant un médicament qui peut être un médicament ionique choisi entre la pseudoéphédrine, la codéine, l'hyrocodone, la phénylpropanolamine, le dextramorphane et l'éphédrine, dans laquelle le médicament est présent sous forme d'un complexe médicament-résine enrobé mis en suspension dans un véhicule liquide, caractérisée en ce que le véhicule contient également de la chlorphéniramine comme second constituant ionique portant la même charge que le médicament complexé enrobé, le second constituant ionique étant présent sous forme d'un complexe non enrobé formé avec la résine.

2. Composition pharmaceutique suivant la revendication 1, dans laquelle le médicament, qui peut être ionique, est la pseudoéphédrine.

3. Composition pharmaceutique suivant la revendication 1, dans laquelle le médicament, qui peut être ionique, est la codéine.

4. Composition pharmaceutique suivant la revendication 1, dans laquelle le médicament, qui peut être ionique, est l'hyrocodone.

**Revendications pour L'Etat contractant: AT**

1. Procédé de préparation d'une composition pharmaceutique à libération prolongée contenant un médicament, qui peut être ionique, choisi entre la pseudoéphédrine, la codéine, l'hyrocodone, la phénylpropanolamine, le dextramorphane et l'éphédrine, dans laquelle le médicament est présent sous forme d'une complexe médicament-résine enrobé mis en suspension dans un véhicule liquide, comprenant les étapes consistant (a) à former une suspension liquide dudit complexe médicament-résine enrobé dans un premier récipient, (b) à former une suspension liquide d'un complexe chlorphéniramine-résine non enrobé dans un second récipient, puis (c) à mélanger les suspensions (a) et (b).

2. Procédé de préparation d'une composition pharmaceutique à libération prolongée contenant un médicament, qui peut être ionique, choisi entre la pseudoéphédrine, la codéine, l'hydrocodone, la phénylpropanolamine, le dextramorphane et l'éphédrine, dans lequel le médicament est présent sous forme d'un complexe médicament-résine enrobé mis en suspension dans un véhicule liquide, qui consiste à mettre en suspension dans un liquide le complexe médicament-résine enrobé et le complexe chlorphéniramine-résine non enrobé.

3. Procédé suivant la revendication 1 ou 2, dans lequel le médicament, qui peut être ionique, est la pseudoéphédrine.

4. Procédé suivant la revendication 1 ou 2, dans lequel le médicament, qui peut être ionique, est la codéine.

5. Procédé suivant la revendication 1 ou 2, dans lequel le médicament, qui peut être ionique, est l'hydrocodone.